# EUROPEAN PATENT APPLICATION

(11) **EP 3 098 601 A1**
(43) Date of publication of application: **30.11.2016**
(21) Application number: 15460071.2
(22) Date of filing: 10.09.2015
(51) Int. Cl.: G01N 33/00

(54) **A SYSTEM FOR PRODUCING REFERENCE GAS MIXTURES, ESPECIALLY SMELL ONES**

(30) Priority: 26.05.2015 PL 41247515
(71) Applicant: Politechnika Gdanska, 80-233 Gdansk (PL)
(72) Inventor: Dymerski, Tomasz, 80-288 Gda?sk (PL); Fija?O, Cyprian, 77-300 Cz?uchów (PL); G?Bicki, Jacek, 80-034 Gda?sk (PL); Namie?NIK, Jacek, 80-299 Gda?sk (PL)

(57) **Abstract**

A system for producing gaseous reference mixtures, especially smell ones, which contains tanks connected with the carrier gas installation and a control system containing a bubbling unit (1), a diffusion unit (2), a permeation unit (3), a dispenser (4) and a chamber for fixing the relative humidity of the carrier gas (5) which at the input are connected in parallel to the carrier gas divider block (6), and at the output are connected in parallel to the mixing chamber (7) which is connected with a pressure compensation tank (8) which is equipped with an output for receiving devices (9). The bubbling unit (1) contains at least one bubbling washer, the diffusion unit (2) contains at least one diffusion chamber and the permeation unit (3) contains at least one permeation chamber. The control system include devices for regulating the intensity of the carrier gas stream. Advantageously, an e-nose device or a tedlar bag or a olfactometric port is the receiving device (9).

## Description

The subject of the invention is a system for producing reference gas mixtures, especially smell ones in a broad spectrum of concentrations, used for calibration of measuring instruments, such as thermal conductivity, magnetic, optical, photocalorimetric gas analysers, gas chromatographs, e-nose devices as well as various gas sensors warning about an explosion or a fire, broadly used in mining, oil processing industry, production of ammonia, urea and other. The produced reference mixtures can also be used in research related to broadly understood environmental protection.

A device for preparing gaseous reference substances which contains a tank for storing the liquid to be determined is known from Polish patent description Patl43557, which on one side has a graduated burette which is connected via an expansion vessel, a valve with a pump on the other side through a capillary tube with a thermal evaporator. The evaporator has stub pipes for supplying gas and collecting the final mixture of steam and gas.

A device and a method of production low gas concentrations in a stream of carrier gas is known from American patent description US 6997347 in which one or more miniature single-part bottles are used which are filled with high-purity gas under pressure or with concentrated gas which is balanced with a neutral gas or a gas mixture. The gas flow is regulated by a pressure regulator or a micro opening. Mixing is continuous to obtain the desired gas concentration. Gas is not generated if the gas pressure in the cylinder which is monitored all the time by the pressure transmitter below the pre-set specific concentration. The device can be built into a portable device or into an automated measuring station and it can be used to calibrate measuring devices.

A method for obtaining gaseous reference mixtures is known from British pattern description GB2040715 in which accurately measured out amounts of gaseous ingredients stored in separate pressurized tanks are mixed using regulation with set of solenoid valves.

On the other hand, a device for producing gaseous reference mixtures is known from the documentation of invention GB850535 in which rotameters, capillary tubes and flow regulators as well as membrane valves are used to regulated the composition of the mixture.

A system for gas permeation at a constant predictable rate is known from patent description US 8720794 which uses amorphous fluoropolymer material in the form of a membrane. This system may include a set of pipes in which gas or liquid are stored. Gas permeation takes place through a membrane in an accurately known amount which results in obtaining constant and known gas concentrations after mixing them with a carrier gas such as air or nitrogen.

A system for automatic regulation of gas permeation is known from patent description US 8327688 in which the control system is connected with a data processor and it allows very precise control of the flow of the pressurized liquid through the system for testing gas permeation.

Many devices are known which are used separately for conducting diffusion, permeation or bubbling processes.

No device is known for producing gaseous reference mixtures in which measured ingredients (analytes) may occur in various concentrations.

A system for producing gaseous reference mixtures, especially smell, which contains connected tanks with a carrier gas installation and a control system, according to the invention, is characterized in that is consists a bubbling unit, a diffusion unit, a permeation unit, a dispenser and a chamber for determining relative humidity of the carrier gas which at the input are connected in parallel to the carrier gas divider block and at the output are connected to the mixing chamber. The mixing chamber is connected to the pressure compensation tank which has an output for receiving devices. The bubbling unit contains at least one bubbling washer, the diffusion unit contains at least one diffusion chamber and the permeation unit contains at least one permeation chamber. The control system includes devices for regulating the temperature and devices for regulating the intensity of the carrier gas stream which the bubbling unit, the diffusion unit, the permeation unit, the dispenser and the chamber for determining relative humidity of the carrier gas are equipped with, devices for regulating the temperature which the carrier gas divider block is equipped with and devices for regulating the carrier gas stream intensity and devices for regulating relative humidity which the chamber for determining relative humidity of the carrier gas and the mixing chamber are equipped with.

Advantageously, an e-nose device or a tedlar bag or a olfactometric port is the receiving device.

The invention presents a solution which contains:
- a unit of bubbling washers, owing to which, as a result of the bubbling process of pure reference substances or their solutions, it is possible to obtain gaseous reference mixtures which are advantageously characterized by specific fragrances with olfactory threshold level at concentrations above 100 ppm v/v,
- a unit of diffusion chambers, owing to which, as a result of the diffusion process of pure reference substances or their solutions, it is possible to obtain gaseous reference mixtures which are advantageously characterized by specific fragrances with olfactory threshold level at concentrations ppb-ppm v/v,
- a unit of chambers with permeation tubes, owing to which, as a result of the permeation process of pure reference substances or their solutions, it is possible to obtain gaseous reference mixtures which are characterized by the the lowest olfactory threshold level at concentrations ppb v/v,
- a dispenser allowing for introducing a specific volume of an odourant or odourants to enhance, replace or reduce the olfactory sensations for the gaseous mixture obtained in the gas chamber. Individual mixtures at specific concentrations obtained
from individual units are mixed in a mixing chamber connected to a concentration compensation tank and the entire process is conducted under temperature stability conditions in a broad range of temperatures 0-60°C for individual streams of gas samples while keeping the temperature gradient between individual units which prevents condensation of steam and analytes.

The system according to the invention can be connected to receiving devices such as e-nose, tedlar bags or an olfactometric port.

The production of gaseous mixtures while keeping constant and specific parameters of temperature and relative humidity guarantees concentrations with high repeatability and reproducible results.

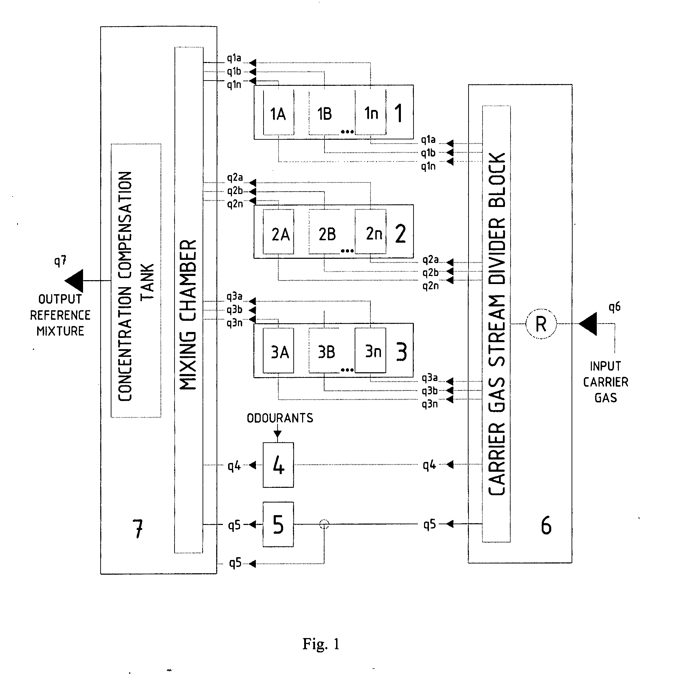

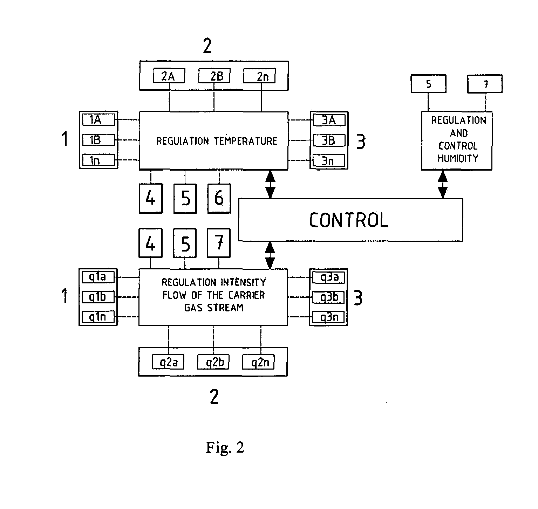

The invention is presented in more detail in the embodiment in the drawing in which Fig. 1 presents a diagram of the system for producing gaseous reference mixtures and Fig. 2 presents a flowchart of the control system.

Volatile organic compounds are used for producing gaseous mixtures of smell compounds such us mercaptans, sulphides, amines, alcohols, ketones, aldehydes, caroboxylic acids and inorganic compounds such as hydrogen sulphide, ammonia. These mixtures are created using bubbling, diffusion and permeation processes. Various compositions of mixtures are obtained by regulating the temperature of the unit of bubbling washers 1, the unit of diffusion chambers 2 and the unit of permeation chambers 3 and regulation of the intensity of the flow of the diluting carrier gas.

The system contains units of independent washers or chambers where bubbling, diffusion and permeation processes occur 1A...n, 2A...n, 3A...n, dispenser 4, carrier gas stream divider 6, mixing chamber 7 and a concentration compensation tank 8, a chamber for determining relative humidity of the carrier gas and for purifying the mixing chamber 5 or the unit for controlling the operation of the device 10 together with appropriate software. Peltier modules were used as devices for regulating the temperature, wires and resistance heaters. The system is equipped with mass flow regulators which are used to regulate the flow of the carrier gas and with humidity sensors which are connected with electric or signal conductors to the central microprocessor controller which is control from the operator panel. It can also be controlled from a PC computer with appropriate software. Each thermostatic chamber of a single washer and the mixing chamber 7 of the gaseous reference mixtures are isolated using insulation materials with possibly the lowest thermal conductivity coefficient.

The system for producing gaseous reference mixtures, especially smell ones, is equipped with a carrier gas stream divider 6 which is reached by zero air from a cylinder or a generator. The pressure and mass intensity of the carrier gas stream flow is regulated using a rotameter and a pressure regulator. The amount of the carrier gas supplied to the units of independent washers and chambers 1A...n, 2A...n, 3A...n in which bubbling, diffusion and permeation processes occur and to the dispenser 4 and the chamber for determining relative humidity of the carrier gas is controlled from the carrier gas stream divider 6: The unit of |washers and chambers 1A...n, 2A...ri, 3A...n is positioned in parallel so that only one duct with the carrier gas is connected with the appropriate washer or chamber from the carrier gas stream divider 6. Each of the chambers and washers is connected at the input to the gas flow mass regulator 6 and at the output to the mixing chamber 7. A channel is situated in parallel to washers and chambers which is equipped with a dispenser 4 which makes it possible to introduce a known amount of an odourant or odourants to enhance or weaken olfactory sensations caused by generated smell mixtures.

The chamber for fixing relative humidity of the carrier gas 5 is positioned in parallel to the unit of washers and chambers 1A...n, 2A...n, 3A...n and the dispenser 4. The humidity fixing module 5 is used for obtaining a constant and specific relative humidity value of the produced gaseous mixture. To determine the constant and specific value of relative humidity, the carrier gas is carried over or through water or a hydrostatic solution as the steam pressure over the specific saturated aqueous solutions of salts of inorganic compounds is constant at a given temperature. The relative humidity of the gaseous mixture in the mixing chamber is determined changing the stream of the carrier gas over or by the hydrostatic solution and by changing the temperature of the chamber containing the hydrostatic solution or water.

To ensure a constant and regulated temperature of the washers and chambers within the range from 0°C to 60°C at which analytes are transferred from the liquid phase to the gaseous phase, the following solutions were used:
- for the temperature range from 0°C to 20°C, thermal regulation using Peltier modules or a cooler,
- for the temperature range from 20°C to 60°C, thermal regulation using Peltier modules or heaters made of resistance wire.

The humidity fixing module 5 contains an isolation valve which is used to change the direction of the carrier gas stream flow to clean the mixing chamber 7 by removing smell mixtures. Streams of gaseous mixtures are directed from the unit of washers and chambers 1A...n, 2A.,.n, 3A...ń, the dispenser 4 and the module for fixing the relative humidity of the carrier gas and cleaning the mixing chamber 5 to the mixing chamber 7 and the concentration compensation tank 8. The temperature of the gaseous mixture in the mixing chamber 7 is regulated using a thermostat which allows the regulation of the temperature within the range from 20°C to approx. 60°C. The use of the thermostatic mixing chamber 7 makes it possible to obtain a constant temperature gradient which prevents the condensation of water and analytes in pneumatic systems.

All devices used for regulation of temperatures, mass flow regulators and hygrometers are connected by means of electric conductors to the central microprocessor controller. The use of such a connection makes it possible to control the operation of the device using the operator panel. The operation of the generator can also be controlled using a personal computer. The output from the mixing chamber 7 allows for simultaneous discharge of the gaseous smell mixture to an e-nose device or a tedlar bag or an olfactometric port.

## Claims

1. A system for producing gaseous reference mixtures, especially smell ones, which contains tanks connected with the carrier gas installation and a control system **characterized in that** it contains a bubbling unit (1), a diffusion unit (2), a permeation unit (3), a dispenser (4) and a chamber for fixing the relative humidity of the carrier gas (5), which at the input are connected in parallel to the carrier gas divider block (6), and at the output are connected in parallel to the mixing chamber (7) which is connected with a pressure compensation tank (8) which is equipped with an output for receiving devices (9), however, the bubbling unit (1) contains at least one bubbling washer, the diffusion unit (2) contains at least one diffusion chamber and the permeation unit (3) contains at least one permeation chamber while the control system includes devices for regulating the temperature and devices for regulating the carrier gas intensity stream which the bubbling unit (1), the diffusion unit (2), the permeation unit (3), the dispenser (4) and the chamber for fixing the relative humidity of the carrier gas (5) are equipped with devices for regulating the temperature which the carrier gas divider block is equipped with (6j and devices for regulating the carrier gas stream and devices for regulating the relative humidity which the chamber for fixing the relative humidity of the carrier gas (5) and the mixing chamber (7) are equipped with.

2. According to claim 1, the system is **characterized in that** an e-nose device or a tedlar bag or a olfactometric port is the receiving device (9).
